# EUROPÄISCHE PATENTSCHRIFT

(11) **EP 1 339 643 B1**
(45) Veröffentlichungstag und Bekanntmachung des Hinweises auf die Patenterteilung: **16.01.2008**
(21) Anmeldenummer: 01993380.3
(22) Anmeldetag: 31.10.2001
(51) Int. Cl.: C02F 1/32, B01J 19/12, A61L 2/10

(54) **VERWENDUNG EINER VORRICHTUNG ZUR BESTRAHLUNG VON FLÜSSIGKEITEN**
USE OF A DEVICE FOR IRRADIATING LIQUIDS
UTILISATION D'UN DISPOSITIF PERMETTANT D'EXPOSER DES LIQUIDES A DES RAYONNEMENTS

(30) Priorität: 13.11.2000 DE 10056096
(43) Veröffentlichungstag der Anmeldung: 03.09.2003
(73) Patentinhaber: Bayer Technology Services GmbH, 51368 Leverkusen (DE)
(72) Erfinder: KAISER, Klaus, 51102 Köln (DE); KAULING, Jörg, 51069 Köln (DE); HENZLER, Hans-Jürgen, 42655 Solingen (DE); GÜNTHER, Isabell, 51373 Leverkusen (DE); SCHMITT, Franz, 51465 Bergisch Gladbach (DE); BECKERS, Erhard, 51399 Burscheid (DE); QUEST, Stefan, 42799 Leverkusen (DE)
(86) Internationale Anmeldenummer: PCT/EP2001/012562
(87) Internationale Veröffentlichungsnummer: WO 2002/038502

(56) Entgegenhaltungen:
- EP-A- 0 202 820
- EP-A- 0 202 891
- WO-A-98/41315
- DE-A- 4 304 444
- DE-A- 4 339 006
- DE-A- 19 719 645
- US-A- 2 636 991
- US-A- 4 769 131
- US-A- 5 785 845

## Beschreibung

Die Erfindung betrifft die Verwendung eines Reaktors zur Einstrahlung von ultraviolettem Licht in ein fluides Reaktionsmedium. Der Reaktor besteht wenigstens aus einem Gehäuse, welches einen rohrförmigen Hohlraum umschließt, mit einer Strahlungsquelle zur Erzeugung von ultraviolettem Licht und einem inneren Rohr, das mit dem Gehäuse einen insbesondere ringförmigen Bestrahlungsraum bildet, wobei der Bestrahlungsraum wenigstens mit einem Einlass und einem Auslass für das Reaktionsmedium verbunden ist und vom Reaktionsmedium in Längsrichtung des Rohres durchströmt wird, und wobei der Bestrahlungsraum Mittel zur Erzeugung einer zusätzlichen radialen Strömungsführung des Reaktionsmediums aufweist.

Die Sterilisation flüssiger Medien ist eine wesentliche Ausgangsvoraussetzung für den Einsatz bio- und lebensmitteltechnologischer Produktionsverfahren. Zielsetzung ist die zuverlässige und vollständige Abreicherung von Mikroorganismen und/oder Viren bei gleichzeitig weitestgehender Erhaltung der Wertstoffe. Sterilisiert werden sowohl die Einsatzstoffe (z.B. Nährmedien für Fermentationen) als auch Endprodukte (z.B. Milchprodukte oder pharmazeutische Wirkstoffproteine). In der Lebensmittelindustrie führen u.a. Aspekte der Haltbarkeitsverlängerung zur Anwendung der Sterilisationstechniken, während deren Einsatz in der pharmazeutischen Industrie durch strenge Qualitätssicherungsauflagen reglementiert ist. So werden zur Anwendung pharmazeutischer Produkte humanen oder tierischen Ursprungs mehrere, auf unterschiedlichen Wirkprinzipien basierende Virusinaktivierungsschritte gefordert, die eine Virusabreicherung um jeweils mindestens vier Zehnerpotenzen gewährleisten. Die Notwendigkeit zur Gewährleistung der "Virussicherheit" trifft selbstverständlich auch auf Pharmazeutika zu, die mit gentechnologischen Verfahren hergestellt werden.

Als produktschonendes Verfahren zur Virusabreicherung wird in der Literatur unter anderem die Bestrahlung mit ultraviolettem Licht vorgeschlagen. Die Behandlung von Plasma und Blutprodukten durch UV-Licht ist grundsätzlich bekannt. Bereits während des zweiten Weltkriegs wurden große Mengen Plasma gesammelt und mit UV-Licht bestrahlt. Die UV-Behandlung von Blutderivativen ist aber besonders hinsichtlich nicht umhüllter, hitzestabiler Viren interessant. Chin et al (Chin, S., Jin, R., Wang, X.L., Hamman, J., Gerard Marx, Xlaode Mou, Inger Andersson, Lars-Olof Lindquist, and Bernhard Horowitz (1997). Virucidal Treatment of Blood Protein Products with UVC Radiation. Photochemistry and Photobiology 65(3): 432-435.) konnten zeigen, dass eine Behandlung von Plasmaprodukten mit UV-Licht zur Inaktivierung von Hepatitis A- und Parvoviren führt.

Zielrichtung der UV-Bestrahlung ist die mutagene Veränderung des Erbmaterials der Mikroorganismen oder Viren, die oberhalb einer Mindestbestrahlungsdosis ihre Vermehrungsfähigkeit verlieren. Aufgabe der Erfindung ist es, hierfür eine sichere und optimal wirksame Vorrichtung zur Bestrahlung mit UV-Licht zu entwickeln.

Probleme bei der Verwendung von Reaktoren zur Einstrahlung von ultraviolettem Licht in flüssige Reaktionsmedien ergeben sich durch eine mit zunehmender Entfernung von der Strahlungsquelle exponentiell abnehmende Strahlungsintensität im zu behandelnden Medium. Mikroorganismen und Viren in einem größeren Abstand von der Strahlenquelle werden aus diesem Grund langsamer bzw. überhaupt nicht mehr abgetötet. Dieser Effekt, der mit zunehmendem Lichtabsorptionsvermögen des Mediums erheblich verstärkt wird, führt nach dem derzeitigen Stand der Technik zur Verwendung sehr großer Bestrahlungsoberflächen, wie man sie z.B. in Dünnschichtreaktoren vorfindet. Die im Einsatz befindlichen Dünnschichtreaktoren lassen sich nur schwer in den technischen Maßstab überführen, da die Konstanthaltung der Filmdicke bei der Maßstabsvergrößerung nur durch eine durchsatzproportionale Durchmesservergrößerung zu realisieren ist, was im technischen Maßstab zu nicht mehr handhabbar großen Reaktoren führt. Einen weiteren negativen Einfluss bildet das ungünstige Verweilzeitverhalten der nach Maßgabe der zumeist nur geringen Eindringtiefe der UV-Strahlung in das Reaktionsmedium notwendigerweise sehr dünnen und damit laminar strömenden Flüssigkeitsfilme, bei denen ein Austausch quer zur Hauptströmungsrichtung definitionsgemäß entfällt. Die wandnahen Schichten verweilen wegen des linear zur Wand bis auf Null abnehmenden Geschwindigkeitsprofiles wesentlich länger als die wandferneren Schichten. Um die zur Abtötung notwendige Mindestbestrahlungsdosis auch in der schneller fließenden wandfemen Flüssigkeitsschicht realisieren zu können, muss die mittlere Verweilzeit des Films angehoben werden. Dies aber führt zu einer erhöhten Strahlenbelastung und somit zur einer größeren Schädigung der Produkte.

Ebenfalls bekannt und beschrieben sind sogenannte Ringspaltreaktoren. Ein UV-Ringspaltreaktor üblicher Bauart besteht aus einem röhrenförmigen Metallgehäuse, in das ein, einen stabförmigen UV-Strahler enthaltendes Quarzrohr eingelassen ist, so dass ein ringspaltförmiger Raum gebildet wird. Bei diesem Reaktortyp fließt das Reaktionsmedium nur in axialer Richtung durch den Ringraum, was im Hinblick auf einen guten Stoffaustausch ähnlich wie bei den Dünnschichtreaktoren ebenfalls nicht vorteilhaft ist.

Die beschriebenen Nachteile der Reaktortypen sollten durch eine günstigere Strömungsführung überwunden werden können, die neben einem engen Verweilzeitspektrum auch einen guten Austausch in der Flüssigkeit senkrecht zur Hauptströmungsrichtung erlauben. Hierzu sind u.a. tangential angeströmte Ringspaltreaktoren vorgeschlagen worden Aus der EP 803 472 A1 ist z.B. ein Reaktor zur Einstrahlung von ultraviolettem Licht in ein Reaktionsmedium mit einem Ringraum als Bestrahlungszone, bei dem der Einlass so ausgebildet ist, dass das Reaktionsmedium tangential in den Ringraum eintritt.

Die Leistung eines Reaktors mit tangentialer Anströmung hat im Vergleich zu einem "klassischen" Ringspaltreaktor marginale Vorteile. Verfahrenstechnische Untersuchungen zeigen, dass das tangentiale Strömungsprofil infolge der Wandreibung bereits kurz nach dem Einlauf in ein axiales Profil umschlägt. Die zumindest für den Bereich der tangentialen Überströmung theoretisch postulierten Deanwirbel, mit denen der Queraustausch des Reaktionsmediums innerhalb des Ringspalts intensiviert werden soll, sind nach visuellen Studien und CFD-Untersuchungen (Strömungssimulation) nicht vorhanden, so dass tangential angeströmte Ringspaltreaktoren dieser Art zwar eine gewisse Verbesserung im Vermischungsverhalten aber dennoch keinen vollständigen Umsatz ermöglichen. Somit ist die Sekundärströmung und der damit verbundene verbesserte Stoffaustausch auf die einlaufnahen Zonen begrenzt.

Es konnte gezeigt werden, dass dieses Verhalten bei der Behandlung schwach absorbierender Reaktionsmedien (z.B. Wasserbehandlung) toleriert werden kann, da hierfür die Vermischung ausreichend ist und die UV-Dosis zur Umgehung dieses Nachteils erhöht werden kann. Für Anwendungen, die in Zusammenhang mit der Behandlung von Proteinlösungen stehen, schien dies nicht möglich zu sein, da die Proteine dabei irreversible Schäden erleiden würden.

Es ist folglich neu und überraschend, dass die Reaktoren der eingangs genannten Art auch für die Behandlung viruskontaminierter Proteinlösungen geeignet sind, wenn der Bestrahlungsraum über seine Länge Mittel zu einer zusätzlichen radialen Strömungsführung des Reaktionsmediums aufweist und insbesondere, wenn in Bezug auf den Durchmesser des Gehäuses eine bestimmte Reaktorlänge nicht überschritten wird. Das vorgeschlagene L/D - Verhältnis sollte bevorzugt kleiner 100 sein.

Wie aus der vorangegangen Diskussion deutlich wird, besteht die Aufgabe der Erfindung darin, Apparate der eingangs genannten Art mit einem optimierten und

Gegenstand der Erfindung ist die Verwendung eines Reaktors zur Einstrahlung von ultraviolettem Licht in ein fluides Reaktionsmedium mit einem Gehäuse, welches einen rohrförmigen Hohlraum umschließt, mit einer Strahlungsquelle zur Erzeugung von ultraviolettem Licht und einem inneren Rohr, das mit dem Gehäuse einen insbesondere ringförmigen Bestrahlungsraum bildet, wobei der Bestrahlungsraum wenigstens mit einem Einlass und einem Auslass für das Reaktionsmedium verbunden ist und vom Reaktionsmedium in Längsrichtung des Rohres durchströmt wird, dadurch gekennzeichnet, dass der Bestrahlungsraum Mittel zur Erzeugung einer zusätzlichen radialen Strömungsführung des Reaktionsmediums aufweist.

Die verwendete Vorrichtung (Reaktor) zur Einstrahlung von UV-Licht in Flüssigkeiten zeichnet sich aufgrund ihres optimalen und gleichmäßigen Vermischungsverhaltens durch einen besseren Stoffaustausch aus, wodurch eine sichere und effektive Sterilisation erreicht wird. Die Vorrichtung lässt sich gut in bestehende Anlagen integrieren und ist einfach zu reinigen. Ebenfalls von Vorteil ist die kompakte Bauweise der Vorrichtung.

Die verwendete Vorrichtung ist dadurch gekennzeichnet, dass in einem für UV-Licht durchlässigen Ringspaltkanal besondere Strömungsbedingungen erzeugt werden, die über die gesamte Kanallänge einen intensiven Stoffaustausch bewirken. Sie besteht z.B. aus einer UV-Strahlenquelle, die von einem Quarzschutzrohr (Strahlerhüllrohr) umgeben sein kann, und einem für UV-Licht durchlässigen Produktkanal (Bestrahlungsraum), durch den das Reaktionsmedium hindurchströmt. Das besondere Kennzeichen des Bestrahlungsraums ist eine intensive, über die gesamte Länge herrschende gleichförmige Quervermischung senkrecht zur Hauptrichtung der Produktströmung sowie eine durch turbulente Produktströmung eingeengte Verweilzeitverteilung.

Durch die Quervermischung wird gewährleistet, dass die von der Strahlenquelle entfernteren Flüssigkeitsschichten, die besonders bei stark lichtabsorbierenden Medien keine oder wenig UV Strahlung erhalten, einen intensiven Austausch mit den UV-bestrahlten Schichten nahe der Strahlenquelle eingehen. Dies wird z.B. durch eine spezielle Strömungsführung in der Vorrichtung erreicht, durch die eine Vielzahl hintereinandergeschalteter, quasi-zellulärer Zirkulationsströmungen erzeugt wird. Hierdurch wird die notwendige Aufenthaltszeit der Produkte in den reaktiven Kanalschichten minimiert, was bei zuverlässiger Sterilisation bzw. Virusinaktivierung zur kleinst möglichen Schädigung der Produkte durch die Strahlenbelastung führt. Die Sekundärströmungen werden beim Durchströmen von spiralförmigen Kanälen erzeugt.

In einer bevorzugten Bauform ist der verwendete Reaktor so gestaltet, dass die Strahlungsquelle im inneren Rohr angeordnet ist und das innere Rohr für das ultraviolette Licht durchlässig ist.

Die Innenwand des Gehäuses weist dabei besonders bevorzugt eine Beschichtung mit einem UV-Strahlung reflektierenden Material auf.

In einer alternativen bevorzugten Bauform ist die Strahlungsquelle des Reaktors außerhalb des Gehäuses angeordnet und das Gehäuse für das ultraviolette Licht durchlässig.

Die Wand des inneren Rohres weist dann besonders bevorzugt eine Beschichtung mit einem UV-Strahlung reflektierenden Material auf.

Aus der Patentschrift US 5 433 738 ist ein Bestrahlungsreaktor für die Bestrahlung von Wasser bekannt, der eine wendelförmige Leitung mit kreisrundem Querschnitt hat. Dieser weist aber keine hinreichende z.B. für die Virusinaktivierung notwendige Quervermischung auf, so dass seine Anwendung für die Virusinaktivierung zu unsicher ist.

Bei den nachfolgend beschriebenen, bevorzugten, kontinuierlich durchflossenen Ringspaltreaktoren mit statischen Einbauten kann auf bewegte Elemente vollständig verzichtet werden. Die Quervermischung kann dabei durch Deanwirbel, Freistrahlen und Produktumschichtung bewirkt werden. Deanwirbel treten in spiralförmigen Rohr- oder Kanalströmungen auf. Es hat sich nun gezeigt, dass die Verwendung von Spiralrohren mit zum Strahler hin abgeflachtem Querschnitt der Rohrflanken, z.B. bei Verwendung von Rechteck- oder D-Profilen, gegenüber den aus dem Stand der Technik bekannten runden Querschnitten zu bevorzugen sind, um eine Schwächung des eingetragenen UV-Lichts durch Lichtreflexionen zu vermeiden.

Bevorzugt ist daher eine Ausführung des Reaktors, bei der das Mittel zur Erzeugung einer zusätzlichen radialen Strömungsführung des Reaktionsmediums und der Bestrahlungsraum durch ein für UV-Strahlung durchlässiges im Querschnitt abgeflachtes Wendelrohr gebildet ist.

Besonders bevorzugt weist das Wendelrohr einen rechteckigen (vorzugsweise mit angerundeten Ecken), ovalen oder halbrunden Querschnitt auf.

Die Anwendung dieser Art von Reaktoren mit Wendelrohr bleibt aber im wesentWendelrohre einer mechanischen Reinigung nicht oder schwer zugänglich sind.

Bevorzugt lassen sich Spiralrohrströmungen auch durch Ausarbeiten von Spiralgängen aus einem von zwei berührungsnah ineinander schiebbaren Zylindern erzeugen.

In einer bevorzugten Variante des Reaktors ist wenigstens ein UV-Sensor mit Messeinrichtung zur Messung der UV-Intensität der Strahlungsquelle am Reaktor angebracht, insbesondere im oberen oder unteren Bereich des Reaktors, z.B. in der Nähe des Einlasses und/oder des Auslasses.

Eine weitere bevorzugte Variante des Reaktors weist wenigstens einen UV-Sensor mit Messeinrichtung zur Messung der UV-Intensität im Bestrahlungsraum, insbesondere im unteren oder oberen Bereich des Reaktors, auf beispielsweise in der Nähe des Einlasses und/oder des Auslasses des Reaktors.

Die Nutzung des Reaktors richtet sich auf eine Vielzahl unterschiedlicher Anwendungen zur UV-Bestrahlung und/oder Sterilisierung von Flüssigkeiten.

Gegenstand der Erfindung ist Verwendung des ober beschriebenen Reaktors zur Bestrahlung und Sterilisierung von Milch- oder Fruchtsanprodukten, chemischen oder pharmazeutischen Produkten, insbesondere bevorzugt von Virus-Vakzinen, gentechnisch erzeugten Wirkstoffen oder Proteinen, z.B. Wirkstoffen oder Proteinen aus transgenen Tieren oder Pflanzen und von Blutplasma oder aus Blutplasma gewonnenen Produkten.

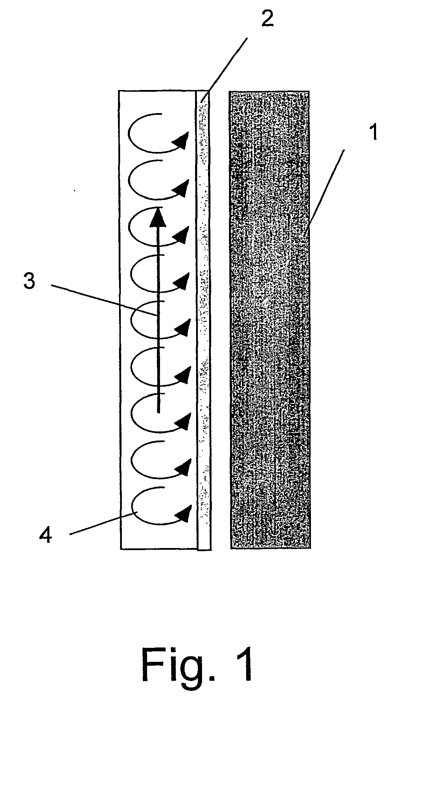

Im folgenden werden Ausführungsbeispiele der Erfindung anhand von Figuren näher erläutert. Es zeigen
- Figur 1: einen schematischen Schnitt durch einen Teil eines Bestrahlungsraums
- Figur 2: einen Ringspaltrührreaktor mit Zylinderrührer, zentrischer UV-Bestrahlung und Magnetantrieb im Längsschnitt
- Figur 2a: ein vergrößertes Detail aus Figur 2
- Figur 3: einen kavernenartig ausgearbeiteten Zylinderrührer aus Figur 2 mit Magnet
- Figur 4a: einen Laufradantrieb für den Zylinderrührer nach Figur 2 im Längsschnitt B-B
- Figur 4b: den Laufradantrieb nach Figur4a im Querschnitt A-A
- Figur 5: einen Wendelrohrreaktor mit halbrundem Rohrquerschnitt
- Figur 5a: ein vergrößertes Detail aus Figur 5
- Figur 6: einen Wendelrohrreaktor mit rechteckigem Rohrquerschnitt
- Figur 6a: ein vergrößertes Detail aus Figur 6
- Figur 7: einen demontierbaren Spiralrohrreaktor mit Kanälen im Längsschnitt
- Figur 7a: ein vergrößertes Detail aus Figur 6
- Figur 8: einen am Innendurchmesser des Ringspaltes angetriebenen Freistrahlreaktor mit trapezförmigen Kanälen im Längsschnitt
- Figur 8a: ein vergrößertes Detail aus Figur 8
- Figur 9: einen Umschichtreaktor mit alternierender Produktzugabe am Innen-und Außendurchmesser des Ringspaltes
- Figur 9a: ein vergrößertes Detail aus Figur 9
- Figur 10: den oberen Teil eines Ringspaltreaktors mit UV-Sensoren

### Beispiele

### Beispiel 1 (nicht erfindungsgemäß)

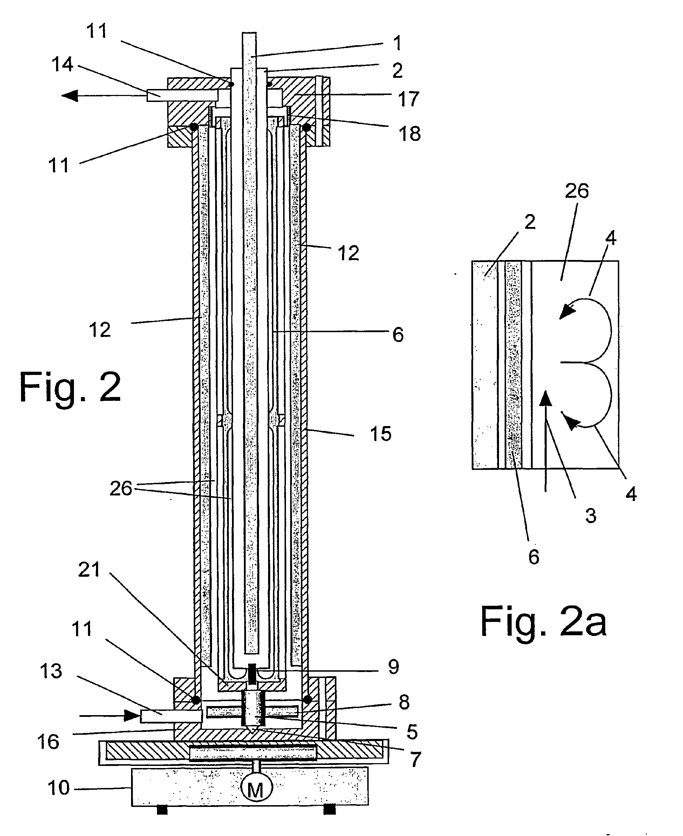

Der UV- Reaktor gemäß Figur 2 und Figur 2a besteht aus einer zentrisch eingebauten UV-Lampe 1 (Hg-Leuchtstoffröhre) mit dem Aussendurchmesser 25 mm und der Länge von 850 mm, die durch einen nach oben geöffneten Quarzglasmantel 2 (inneres Quarzglasrohr) mit Innendurchmesser 26 mm ohne Produktberührung eingeführt und entnommen werden kann. Die offene Seite des Rohres 2 ist durch einen O-Ring 11 im Kopfdeckel 17 steriltechnisch einwandfrei gedichtet. Um das Glasrohr 2 rotiert in geringem Abstand von 0,5 mm ein mit 8 Rührblättern bewehrter Zylindersicher 6. Dieser wird im Kopfdeckel 17 durch ein Gleitlager 18 und im Bodendeckel 16 durch eine Zentrierspitze 7 gelagert. Der Antrieb erfolgt durch Magnetkupplung, indem die von einem externen Magnetrührwerk 10 zur Verfügung gestellte Leistung auf einen quer zur Rührwelle 5 montierten Gegenmagneten 8 berührungs- und somit dichtungslos übertragen wird. Zur Gewährleistung des Wandabstandes zwischen Zylinderrührer 6 und Glasrohr 2 wird die achsensymmetrische Lage des Rohres 2 mittels eines Zentrierstiftes 9 gesichert, der innerhalb der Welle 5 geführt wird. Der Ringspalt 26 (Bestrahlungsraum) von 5 mm, in dem die Inaktivierungsreaktion stattfindet, wird nach innen begrenzt durch die Außenwand des Rohres 2 und nach außen durch die optional mit vier 3 mm breiten Strombrechern 12 bewehrte Innenwand des Mantelrohres 15, an dessen beiden Enden Flansche zur Befestigung des Boden- 16 und den Kopfdeekels 17 angeschweißt sind.

Das Produkt wird mit einem Durchsatz von 150 - 300 l/h in den Stutzen 13 des Bodendeckels eingespeist und am Kopfdeckel 17 über Stutzen 14 abgezogen.

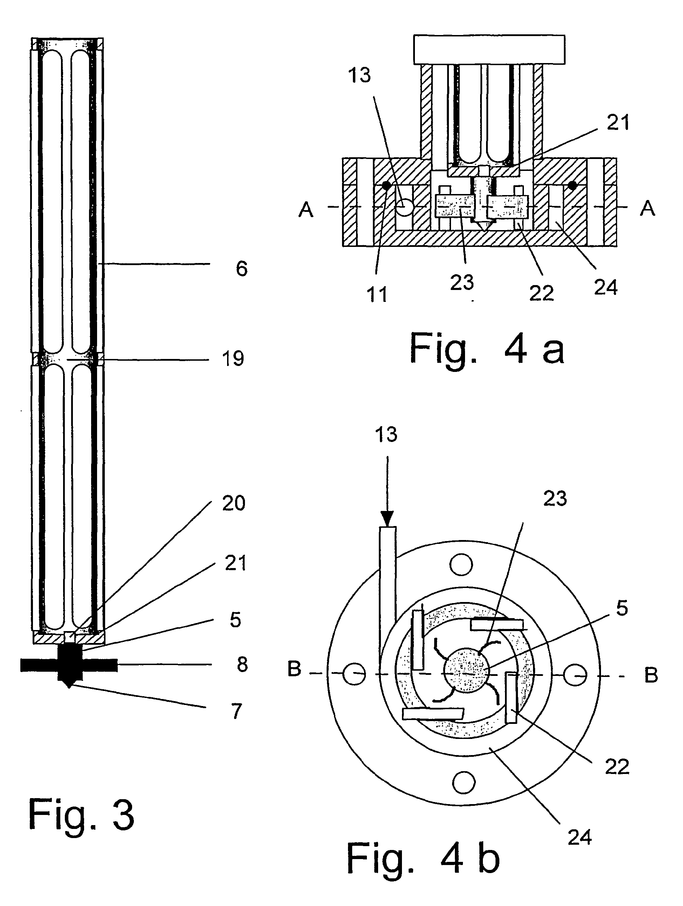

Der Zylinderrührer 6 mit 8 Blättern in der Form gemäß Figur 3 wird aus einem Präzisionsrohr mit dem Außendurchmesser 31 mm und der Wandstärke 0,8 mm durch Ausschneiden 16 kavernenartiger Aussparungen angefertigt. Die Aussparungen erstrecken sich aus Stabilitätsgründen nicht durchgängig über die gesamte Rührerlänge, sondern reichen bis zu den Stegverbindungen 19. Der Ankerrührer 6 ist über eine Scheibe 21 mit der Rührwelle 5 verbunden. Die bis in die Welle 5 hineinreichende Zentrieröffnung 20 dient zur Zentrierung des unten geschlossenen Rohres 2. Der Rührer seinerseits wird im Bodendeckel 16 über eine Zentrierspitze 7 zentriert.

### Beispiel 2 (nicht erfindungsgemäß)

Der als Alternative zur Magnetkupplung in der Bauform nach Figur 2 eingesetzte ebenfalls dichtungslose Schaufelradantrieb für den Zylinderrührer 6 gemäß der Figur 4a (Schnitt B-B) und 4b (Schnitt A-A) besteht aus 4 auf der Rührwelle 5 befestigten, konvex gebogenen Schaufelrädern 23 mit einer Höhe von 10 mm. Der Durchmesser des Schaufelrades beträgt 39 mm. Der Antrieb erfolgt durch das in einen vorgelagerten Ringraum 24 tangential eingeleitete Produkt, das über vier gegen den Innenraum tangential angestellte Spalte 22 mit einer Spaltweite von jeweils 0,8 mm auf die Antriebsschaufeln geleitet wird.

### Beispiel 3

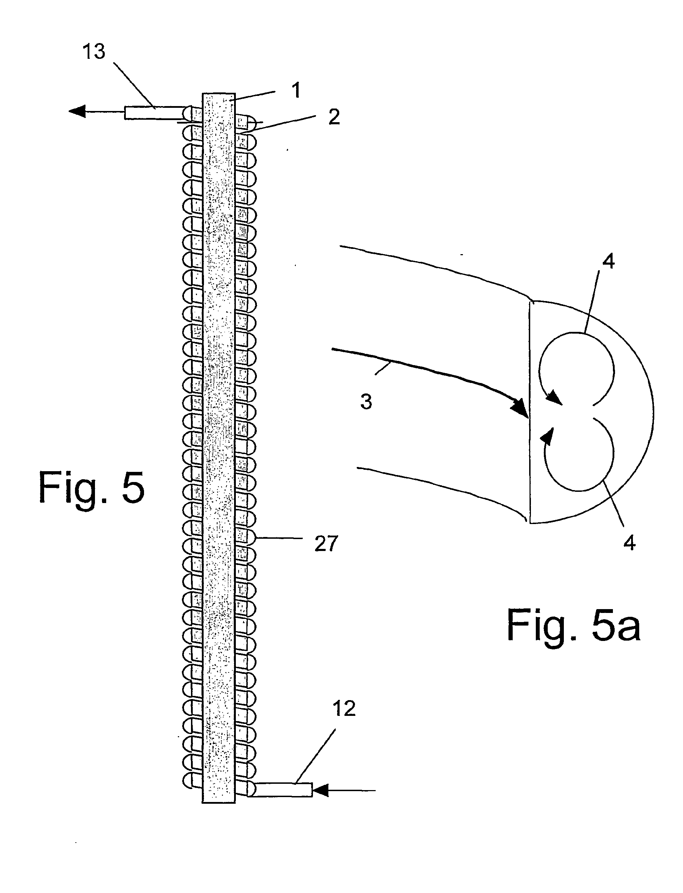

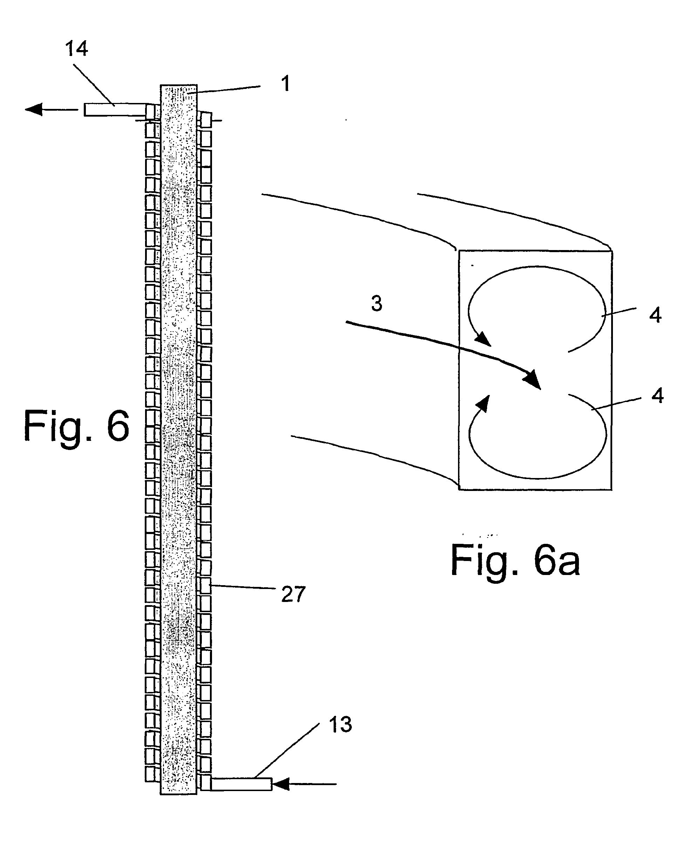

Der UV- Reaktor gemäß Figur 5 und Figur 5a besteht aus einer UV-Lampe 1 mit dem Durchmesser 25 mm, um die ein Quarzglasrohr 27 mit halbrundem Querschnitt von 8 mm Durchmesser und 4 mm Radius in geringem Abstand gewickelt ist. Der UV- Reaktor gemäß Figur 6 und Figur 6a besteht aus einer UV-Lampe 1 mit dem Durchmesser 25 mm, um die ein Quarzglasrohr 27 mit rechteckigem Querschnitt von 8 mm Breite und 4 mm Tiefe in geringem Abstand gewickelt ist. Rechtwinklig zur Spiralströmung 3 werden Sekundärwirbel 4, sog. Dean-Wirbel induziert, die eine Umwälzung des zu bestrahlenden Gutes im Rohr 27 zur Folge haben.

### Beispiel 4 (nicht erfindungsgemäß)

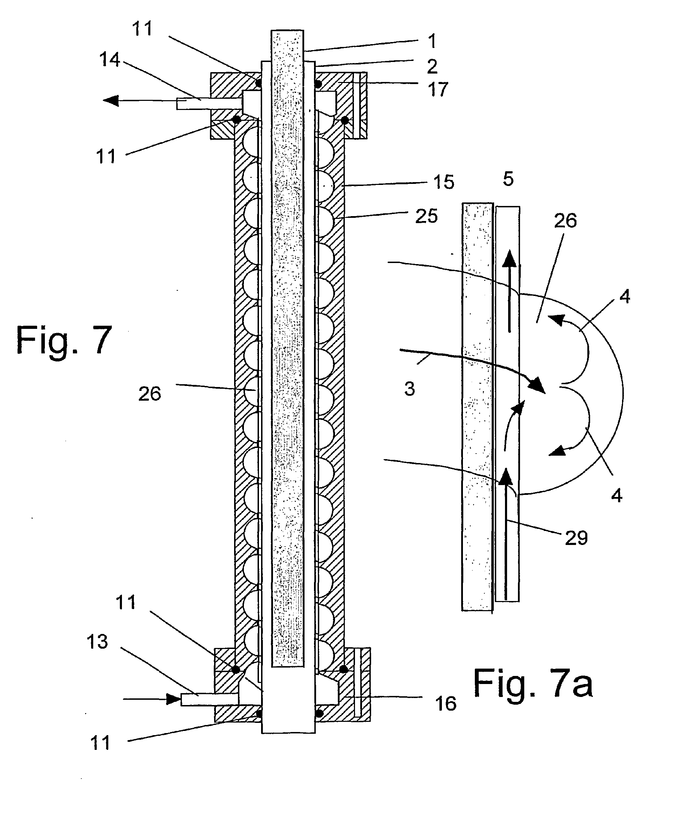

Der UV-Reaktor gemäß Figur 7 und Figur 7a besteht aus einem UV-Strahler 1 mit umgebendem, durchgängigem, am Kopf- 17 und Bodendeckel 16 mit O-Ringen 11 gegen den Reaktionsraum 26 gedichtetem Quarzglasrohr 2, das auf der Außenseite spiralförmig vom Produkt überströmt wird. Die Kontur der spiralförmigen Strömung wird durch den Kanal 25 im Außenzylinder 15 vorgegeben. Zur Erzeugung des Kanals 25 ist in den Außenzylinder 15 mit einer Tiefe von 4 mm und einer Breite von 6 mm ein Rundgewindegang eingeschnitten sind. Der kleinste Abstand zwischen Glasrohr 2 und Zylinder beträgt 0,5 mm. Dieser Spalt erlaubt zur Verringerung der Fouling-Problematik einem Teil der Produktströmung als Freistrahl 29 in den darüber liegenden Strömungskanal einzutreten. Die nahezu senkrecht auf die spiralförmige Hauptströmung 3 gerichtete Freistrahlströmung 29 (siehe Figur 7a) führt zu einer weiteren Verstärkung des ersten der beiden durch die Spiralströmung erzeugten Sekundärwirbel 4. Mit dieser Anordnung wird eine gegenüber dem Wendelrohr deutlich verbesserte gleichmäßige Bestrahlung des Produktes erreicht.

### Beispiel 5 (nicht erfindungsgemäß)

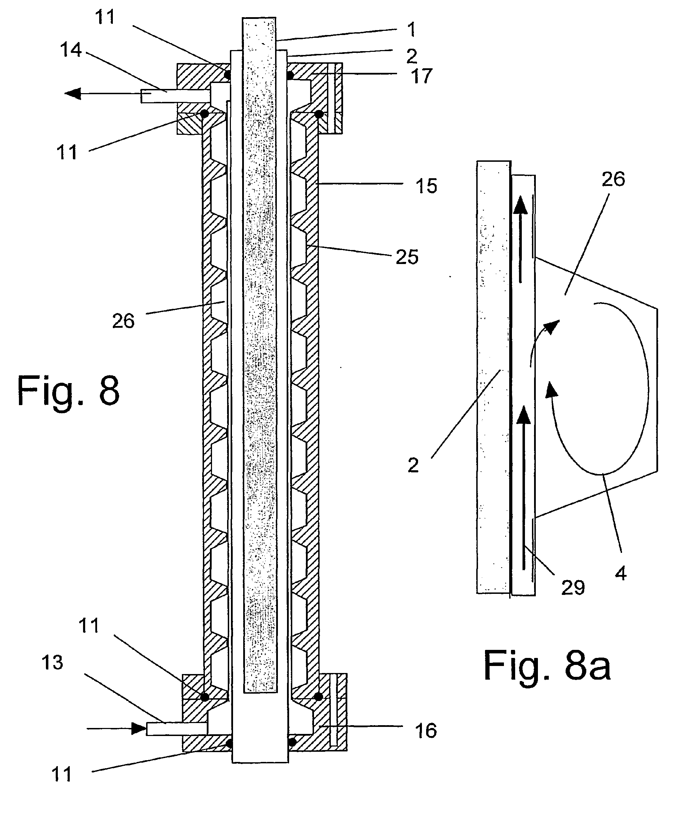

Der UV-Reaktor gemäß Figur 8 und Figur 8a besteht aus einem UV-Strahler 1 mit umgebendem, durchgängigem, am Kopf- 17 und Bodendeckel 16 mit O-Ringen 11 gegen den Reaktionsraum 26 gedichtetem Quarzglasrohr 2, das auf der Produktseite in axialer Richtung überströmt wird. Durch die sägezahnförmige Kontur der Kanäle 25° des Außenzylinders 15 werden eine Vielzahl voneinander abgegrenzter Ringspalträume mit einer Höhe von 30 mm und einer Spaltweite von 4 mm für die Ausbildung der Sekundärwirbel 4 geschaffen (vergl. Figur 8a). Die Sekundärwirbel werden durch Freistrahlen 29 angetrieben, die im 0,7 mm breiten Ringspalt beim Eintritt des Produkstromes in die Kanäle 25' erzeugt werden.

### Beispiel 6 (nicht erfindungsgemäß)

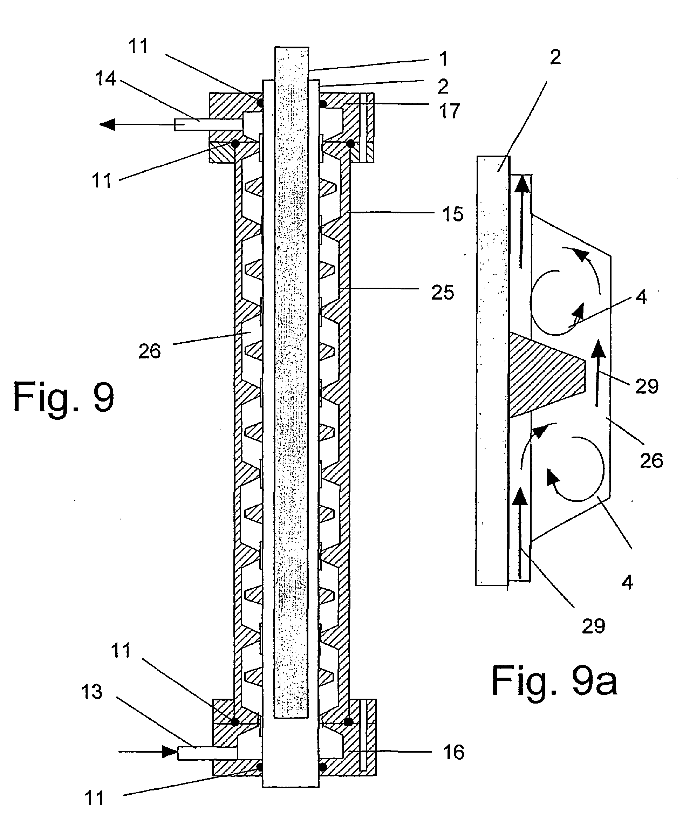

Der UV-Reaktor gemäß Figur 9 und Figur 9a besteht aus einem UV-Strahler 1 mit umgebendem, durchgängigem, am Kopf- 17 und Bodendeckel 16 mit O-Ringen 11 gegen den Reaktionsraum 26 gedichtetem Quarzglasrohr 2, das auf der Produktseite in axialer Richtung überströmt wird. Durch die spezielle Kontur der Kanäle 25' des Außenzylinders werden Kammern mit einer Breite von 4 mm und einer Höhe von 30 mm gebildet, in denen mit Freistrahlen 61 angetriebene gegenläufige Sekundärwirbel 4 erzeugt werden, die zu einer alternierenden Umschichtung des Produktes von der Innenseite zur Außenseite der Kammern führen. Die axialen Freistrahlen werden in 0,7 mm breiten Ringspalten auf dem inneren und äußeren Umfang der Kammern erzeugt.

### Beispiel 7

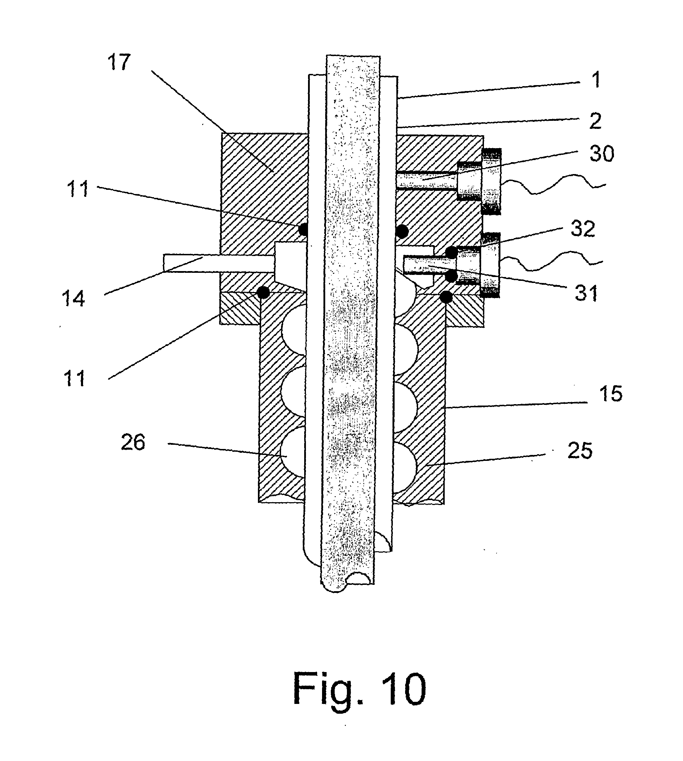

Der UV-Reaktor gemäß Figur 10 (Teilansicht) ist gegenüber dem Reaktor nach Figur 7 dahingehend modifiziert, dass der Spalt zwischen Glasrohr 2 und Außenzylinder 15 weggelassen ist.

Zusätzlich ist im Kopfbereich 17 ein UV-Sensor 30 angebracht, der direkt die von dem UV-Strahler 1 abgegebene UV-Strahlung misst. Hiermit wird z.B. eine Regelung der UV-Intensität ermöglicht.

Ein zweiter UV-Sensor 31 ist im Bestrahlungsraum angeordnet, um "Fouling-Prozesse" im Reaktor beobachten zu können.

Am Fuß des Reaktors befinden sich weitere zwei UV-Sensoren für die prinzipiell gleichen oben angegebenen Zwecke (in Figur 10 nicht gezeichnet).

## Patentansprüche

1. Verwendung eines Reaktors zur Bestrahlung und Sterilisierung von Milch- oder Fruchtsaftprodukten, chemischen oder pharmazeutischen Produkten, insbesondere bevorzugt von Virus-Vakzinen, gentechnisch erzeugten Wirkstoffen oder Proteinen, Wirkstoffen oder Proteinen aus transgenen Tieren oder Pflanzen und von Blutplasma oder aus Blutplasma gewonnenen Produkten, wobei der Reaktor ein Gehäuse (15) aufweist,
welches einen rohrförmigen Hohlraum umschließt, mit einer Strahlungsquelle (1) zur Erzeugung von ultraviolettem. Licht und einem inneren Rohr (2), das mit dem Gehäuse (15) einen insbesondere ringförmigen Bestrahlunsgsraum (26) bildet wobei der Bestrahlungsraum (26) wenigstens mit einem Einlass (13) und einem Auslass (14) für die zu bestrahlende Flüssigkeit (3) verbunden ist und der zu bestrahlenden Flüssigkeit (3) in Längsrichtung des Rohres (2) durchströmt wird, **dadurch gekennzeichnet, dass** der Bestrahlungsraum (26) ein Element (6, 25) zur Erzeugung einer zusätzlichen radialen Strömungsführung des Reaktionsmediums (3) enthält, wobei das Element ein für UV-Strahlung durchlässiges Wendelrohr mit zum Strahler hin abgeflachtern Querschnitt des Rohrflanken mit D-förmigem, halbrundem oder vorzugsweise mit abgerundeten Ecken versehenen, rechteckigen Querschnittsprofil und gleichzeitig Bestrahlungsraum (26) ist.

2. Verwendung nach Anspruch 1, **dadurch gekennzeichnet, dass** die Strahlungsquelle (1) im inneren Rohr (2) angeordnet ist und das innere Rohr (2) für das ultraviolette Licht durchlässig ist.

3. Verwendung nach Anspruch 2, **dadurch gekennzeichnet, dass** die Innenwand des Gehäuses (15) eine Beschichtung mit einem UV-Strahlung reflektierenden Material aufweist.

4. Verwendung nach Anspruch 1, **dadurch gekennzeichnet, dass** die Strahlungsquelle (1) außerhalb des Gehäuses (15) angeordnet ist und das Gehäuse (15) für das ultraviolette Licht durchlässig ist.

5. Verwendung nach Anspruch 4, **dadurch gekennzeichnet, dass** die Wand des inneren Rohres (2) eine Beschichtung mit einem UV-Strahlung reflektierenden Material aufweist.

6. Verwendung nach einem der Ansprüche 1 bis 5, **dadurch gekennzeichnet, dass** wenigstens ein UV-Sensor (30) mit Messeinrichtung zur Messung der UV-Intensität der Strahlungsquelle (1) am Reaktor angebracht ist, insbesondere im oberen oder unteren Bereich des Reaktors.

7. Verwendung nach einem der Ansprüche 1 bis 6, **dadurch gekennzeichnet, dass** wenigstens ein UV-Sensor (31) mit Messeinrichtung zur Messung der UV-Intensität im Bestrahlungsraum (26), insbesondere im unteren oder oberen Bereich des Reaktors, angebracht ist.

## Claims

1. Use of a reactor for irradiating and sterilising milk or fruit juice products, chemical or pharmaceutical products, particularly preferably virus vaccines, active substances or proteins produced by genetic engineering, active substances or proteins from transgenic animals or plants, and blood plasma or products obtained from blood plasma, wherein the reactor comprises a housing (15), which encloses a tubular hollow space, with a radiation source (1) for generating ultraviolet light and an inner tube (2) which, together with the housing (15), forms an, in particular, annular-shaped irradiation chamber (26), wherein the irradiation chamber (26) is connected to at least one inlet (13) and at least one outlet (14) for the liquid (3) to be irradiated, and the liquid (3) to be irradiated flows through the irradiation chamber in the longitudinal direction of the tube (2), **characterised in that** the irradiation chamber (26) contains an element (6, 25) for generating an additional radial flow guidance of the reaction medium (3), wherein the element is a helical tube, permeable to UV radiation, with a cross-section of the tube side flattened with respect to the radiator and with a D-shaped, semi-round cross-sectional profile or a rectangular cross-sectional profile preferably provided with rounded edges, and at the same time serves as irradiation chamber (26).

2. Use according to claim 1, **characterised in that** the radiation source (1) is arranged in the inner tube (2) and the said inner tube (2) is permeable to ultraviolet light.

3. Use according to claim 2, **characterised in that** the inner wall of the housing (15) has a coating of a material reflecting UV radiation.

4. Use according to claim 1, **characterised in that** the radiation source (1) is arranged outside the housing (15) and the housing (15) is permeable to ultraviolet light.

5. Use according to claim 4, **characterised in that** the wall of the inner tube (2) has a coating with a material reflecting UV radiation.

6. Use according to one of claims 1 to 5, **characterised in that** at least one UV sensor (30) with a measuring device for measuring the UV intensity of the radiation source (1) is mounted on the reactor, in particular in the upper or lower region of the reactor.

7. Use according to one of claims 1 to 6, **characterised in that** at least one UV sensor (31) with a measuring device for measuring the UV intensity in the radiation chamber (26) is mounted in particular in the lower or upper region of the reactor.

## Revendications

1. Utilisation d'un réacteur pour irradier et stériliser des produits dérivés du lait ou de jus de fruit, des produits chimiques ou pharmaceutiques, notamment et de préférence des vaccins contre des virus, des agents actifs ou des protéines produits par une technique génétique, des agents actifs ou des protéines issus d'animaux ou de plantes transgéniques et du plasma sanguin ou des produits obtenus à partir du plasma sanguin, le réacteur comportant un carter (15) qui enveloppe un espace creux tubulaire, avec une source de rayonnement (1) pour la production de lumière ultraviolette et avec un tube intérieur (2) qui forme avec le carter (15) un espace d'irradiation (26) notamment tubulaire, l'espace d'irradiation (26) étant relié à au moins un orifice d'admission (13) et un orifice d'évacuation (14) pour le liquide à irradier (3) et le liquide à irradier (3) traversant le tube (2) dans le sens de sa longueur, **caractérisée par le fait que** l'espace d'irradiation (26) contient un élément (6, 25) pour la production d'un guidage d'écoulement radial supplémentaire du fluide de réaction (3), l'élément étant un tube hélicoïdal qui est transparent au rayonnement UV, qui a une section transversale du flanc de tube aplatie en direction du dispositif de rayonnement et qui a un profil de section transversale en forme de D, en demi-rond ou de forme rectangulaire munie de préférence d'angles arrondis et est simultanément un espace d'irradiation (26).

2. Utilisation selon la revendication 1, **caractérisée par le fait que** la source de rayonnement (1) est placée dans le tube intérieur (2) et que le tube intérieur (2) est transparent à la lumière ultraviolette.

3. Utilisation selon la revendication 2, **caractérisée par le fait que** la paroi intérieure du carter (15) comporte un revêtement avec un matériau réfléchissant le rayonnement UV.

4. Utilisation selon la revendication 1, **caractérisée par le fait que** la source de rayonnement (1) est placée en dehors du carter (15) et que le carter (15) est transparent à la lumière ultraviolette.

5. Utilisation selon la revendication 4, **caractérisée par le fait que** la paroi du tube intérieur (2) comporte un revêtement avec un matériau réfléchissant le rayonnement UV.

6. Utilisation selon l'une des revendications 1 à 5, **caractérisée par le fait qu'**au moins un capteur UV (30) avec dispositif de mesure pour mesurer l'intensité UV de la source de rayonnement (1) est placé sur le réacteur, notamment dans la zone supérieure ou inférieure du réacteur.

7. Utilisation selon l'une des revendications 1 à 6, **caractérisée par le fait qu'**au moins un capteur UV (31) avec dispositif de mesure pour mesurer l'intensité UV est placé dans l'espace d'irradiation (26), notamment dans la zone inférieure ou supérieure du réacteur.
